# EUROPEAN PATENT SPECIFICATION

(11) **EP 2 740 462 B1**
(45) Date of publication and mention of the grant of the patent: **10.10.2018**
(21) Application number: 12820272.8
(22) Date of filing: 24.07.2012
(51) Int. Cl.: A61K 8/06, A61K 8/25, A61K 8/64, A61K 8/81, A61Q 17/04, A61Q 19/00

(54) **OIL-IN-WATER-TYPE EMULSION COSMETIC**
ÖL-IN-WASSER-EMULSIONSKOSMETIKUM
COSMÉTIQUE SOUS FORME D'ÉMULSION DE TYPE HUILE-DANS-EAU

(30) Priority: 02.08.2011 JP 2011169382
(43) Date of publication of application: 11.06.2014
(60) Divisional application: 16152752.8
(73) Proprietor: Shiseido Company, Ltd., Tokyo 104-0061 (JP)
(72) Inventor: SATO, Tomoko, Yokohama-shi Kanagawa 224-8558 (JP); TESHIGAWARA, Takashi, Yokohama-shi Kanagawa 224-8558 (JP); REGER, Martin, 95448 Bayreuth (DE); HOFFMANN, Heinz, 95448 Bayreuth (DE); SUGIYAMA, Yuki, Yokohama-shi Kanagawa 224-8558 (JP); KITAJIMA, Masaki, Yokohama-shi Kanagawa 224-8558 (JP)
(74) Representative: Lippert Stachow Patentanwälte Rechtsanwälte
(86) International application number: PCT/JP2012/068692
(87) International publication number: WO 2013/018584

(56) References cited:
- EP-A2- 1 430 906
- WO-A1-2010/098099
- WO-A1-2010/113930
- WO-A1-2011/069674
- DE-A1- 10 124 914
- JP-A- 59 020 212
- JP-A- 2000 095 638
- JP-A- 2001 518 111
- JP-A- 2006 257 021
- JP-A- 2008 074 779
- US-A- 5 788 955
- US-A1- 2009 136 433
- "Improvement of skin sensory of cosmetic emulsions containing particulate micronized insoluble organic UV filters agents insoluble therein and / or insoluble micronized inorganic UV filters", IP.COM JOURNAL, IP.COM INC., WEST HENRIETTA, NY, US, 1 September 2009 (2009-09-01), XP013133739, ISSN: 1533-0001
- "Suncare compositions with new cosmetic raw materials", IP.COM JOURNAL, IP.COM INC., WEST HENRIETTA, NY, US, 2 March 2010 (2010-03-02), XP013137210, ISSN: 1533-0001
- JUTZ, G ET AL.: 'Bio-inorganic microcapsules from templating protein- and bionanoparticle-stabilized Pickering emulsions' JOURNAL OF MATERIALS CHEMISTRY vol. 20, no. 21, 2010, pages 4299 - 4304, XP055142317

## Description

### RELATED APPLICATIONS

This application claims the priority of Japanese Patent Application No. 2011-169382 filed on August 2, 2011.

### FIELD OF THE INVENTION

The present invention relates to an oil-in-water emulsion cosmetic, and in particular, relates to the oil-in-water emulsion cosmetic that have excellent in emulsion stability, temporal emulsion stability, and feeling in use such as the free of stickiness.

### BACKGROUND OF THE INVENTION

In many cases, emulsion cosmetics have aqueous components and oil components stably mixed by the emulsification action of added surfactants.

Meanwhile, as consumers placing greater importance on safety have increased in recent years, their demands have increased for emulsion cosmetics comprising a surfactant, which might cause skin irritation in an extremely sensitive user in rare cases or may generate stickiness, in no or a low enough content to give no such skin irritation.

The emulsions prepared by using no surfactant but by allowing powder to be adsorbed in the interface have been conventionally known as Pickering emulsions. A large number of research results have been reported up to now on the preparation of Pickering emulsions (for example, refer to Non-Patent literature 1), and a practical use thereof has been proposed in the field of cosmetics (for example, refer to Patent literature 1).

Other than the powder, some reports have also been made on the application of Pickering emulsion-like emulsions to cosmetics by using vesicles, polymer aggregates or hydrophobin derivatives which are proteins, as a material that does not dissolve in aqueous phase and oil phase (for example, refer to Patent literatures 2 to 4).

However, it has been very difficult to prepare Pickering emulsions having stability against temperature and stirring in various environments.

In addition, as for the emulsion described in Patent literature 4, some thickeners are substantially essential to provide the stability of the emulsion, but the thickeners may cause sticky or slimy feelings in use. Another problem is that the emulsion causes increase in viscosity temporally when emulsified with a hydrophobin derivative.

In this way, although there have been reported techniques for comprising an amphiphilic agent which does not dissolve in the aqueous phase and the oil phase so far for the purpose of obtaining stable emulsions, it has been difficult to obtain an oil-in-water emulsion cosmetics having sufficient stability. In addition, a new problem of the feeling in use such as sticky feeling of the cosmetic due to an amphiphilic agent has also occurred.

Patent literature 1: Japanese Unexamined Patent Publication No. 2001-518111
Patent literature 2: Japanese Unexamined Patent Publication No. 2006-239666
Patent literature 3: Japanese Patent No. 3549995
Patent literature 4: Japanese Unexamined Patent Publication No. 2009-501701

EP 1430906 A2 discloses an oil-in-water emulsion cosmetic comprising an acrylamide methylpropane sulfonic acid - vinylpyrrolidone - copolymere, particles with the average particle size of less than 500 nm in form of titanium dioxide particles, an oil component and an aqueous component.

IP.com Journal, September 1, 2009, XP 013133739 and IP.com Journal, March 2, 2010, XP 013137210 disclose suncare compositions comprising acrylamide methylpropane sulfonic acid - vinylpyrrolidone - copolymere and UV-absorber particles.

WO 2011/069674 A1 discloses a foam formulation comprising a substantially emulsifier-free O/W emulsion comprising acrylamide methylpropane sulfonic acid vinylpyrrolidone - copolymer.

Non-patent literature 1: B. Binks et. Al, Advances in Colloid and Interface Science 100-102 (2003)

### DISCLOSURE OF THE INVENTION

### PROBLEM TO BE SOLVED BY THE INVENTION

The present invention was made in view of the above-described problems, and an object of the invention is to provide an oil-in-water emulsion cosmetic having excellent in emulsion stability, temporal emulsion stability and feeling in use such as the free of stickiness although the cosmetic do not substantially comprise a surfactant.

### MEANS TO SOLVE THE PROBLEM

In order to achieve the above-described object, the present inventors have conducted a study. As a result, the present inventors have found that an oil-in-water emulsion cosmetic according to claim 1 in which a specific amphipathic substance and particles with the average particle size of less than 500 nm are comprised in combination has excellent in emulsion stability, temporal emulsion stability and feeling in use, thus leading to completion of the present invention.

That is, the oil-in-water emulsion cosmetic of the present invention is characterized by comprising the following components (a) to (d):
(a) an amphipathic substance being a copolymer of 2-acrylamido-2methylpropane sulfonic acid or a salt thereof and vinylpyrrolidone,
(b) particles with the average particle size of less than 500 nm, wherein component (b) is one or more kinds selected from smectites, fluorine mica and boehmite,
(c) an oil component, and
(d) an aqueous component,
wherein the blending quantity of a surfactant is less than 0.5 mass % of the total amount of the cosmetic.

In the cosmetic, it is preferable that component (a2) is represented by the following general formula (1). 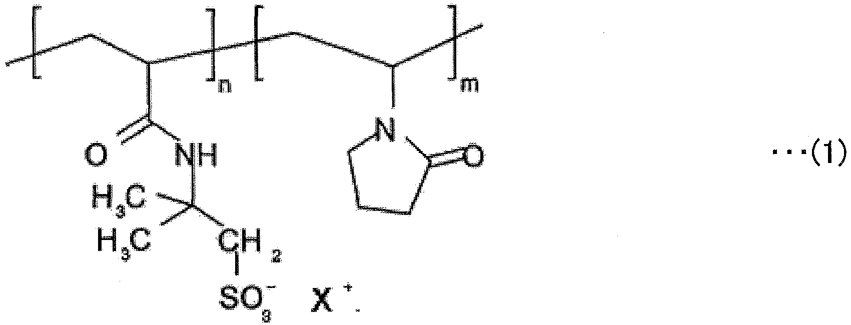 (In the formula, m and n are average addition mole numbers, and X⁺ represents a proton, an alkali metal cation, an alkaline earth metal cation, an ammonium ion, or an organic cation.)

In the cosmetic, it is preferable that the blending quantity of component (c) is 5 to 70 mass % of the total amount of the cosmetic.

In the cosmetic, it is preferable that the blending mass ratio of component (a) and component (b) is 1:20 to 2:1.

In the cosmetic, it is preferable that the blending quantity of a thickener is 0.05 mass % or less of the total amount of the cosmetic.

### EFFECT OF THE INVENTION

The oil-in-water emulsion cosmetic of the present invention according to claim 1 comprises (a) specific amphipathic substance, (b) particles with the average particle size of less than 500 nm, (c) an oil component, and (d) an aqueous component, and it has an emulsion stability, temporal emulsion stability, and excellent in feeling in use.

### BEST MODE FOR CARRYING OUT THE INVENTION

The oil-in-water emulsion cosmetic of the present invention comprises (a) an amphipathic substances being a copolymer of 2-acrylamido-2methylpropane sulfonic acid or a salt thereof and vinylpyrrolidone, (b) particles with the average particle size of less than 500 nm, wherein component (b) is one or more kinds selected from smectites, fluorine mica and boehmite, (c) an oil component, and (d) an aqueous component, and wherein the blending quantity of a surfactant is less than 0.5 mass % of the total amount of the cosmetic.

In the following, each component is described in detail.

### (a) Amphipathic substance

(a) An amphipathic substance incorporated in the oil-in-water emulsion cosmetic of the present invention is a copolymer of 2-acrylamido-2methylpropane sulfonic acid or a salt thereof and vinylpyrrolidone.

The copolymer (a) 2-acrylamido-2-methylpropane sulfonic acid or a salt thereof/vinylpyrrolidone is obtained by the copolymerization of the building block 2-acrylamido-2-methylpropane sulfonic acid or a salt thereof and the building block vinylpyrrolidone; the copolymer may or may not be crosslinked.

The component (a) is preferably a compound represented by the following general formula (1).

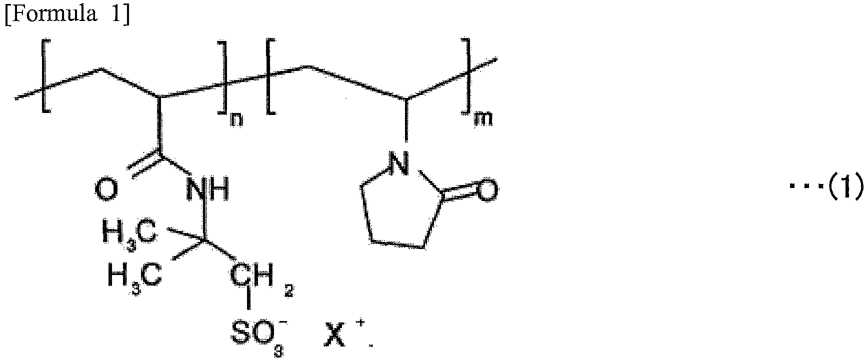

In the general formula (1), m and n are average addition mole numbers, respectively. It is preferable that m and n satisfy m≧1 and n≧1, respectively. m and n satisfy preferably 5000≦m+n≦1000000 and more preferably 10000≦m+n≦500000, respectively.

X⁺ represents a proton, an alkali metal cation (for example, Na⁺ and K⁺), an alkaline earth metal cation (for example, Ca⁺⁺ and Mg⁺⁺), an ammonium ion (NH₄⁺), or an organic cation (for example, n-alkyl ammonium ion and n-alkanol ammonium ion).

The molecular weight of compound represented by the general formula (1) is preferably 500,000 or higher and more preferably 1,000,000 or higher. The molecular weight is preferably 5,000,000 or less and more preferably 20,000,000 or less.

The production method of component (a) is not limited in particular, and it can be produced by the conventional method.

As component (a), (sodium acryloyl dimethyl taurine/VP) copolymer is preferably used. As (sodium acryloyl dimethyl taurine/VP) copolymer, a commercial product (for example, Aristoflex AVC (manufactured by Clariant UK)) can be used.

The blending quantity of (a) amphipathic substance is preferably 0.01 to 1 mass % of the total cosmetic. The blending quantity of component (a) is especially preferably 0.1 mass % or higher. If it is too small, the emulsion stability may be poor. The blending quantity of component (a) is especially preferably 0.5 mass % or less. If it is too large, the stickiness is high and the scent may not be favorable.

### ((b) Particles with the average particle size of less than 500 nm)

For (b) particles with the average particle size of less than 500 nm that is incorporated in the oil-in-water emulsion cosmetics of the present invention, the particles usually used in cosmetics can be incorporated in such a range that the stability of the emulsion is not deteriorated.

Increase in viscosity by temporal aggregation of emulsion particles and network formation of the component (a) can be inhibited by incorporating particles with the average particle size of less than 500 nm in the cosmetics of the present invention.

Examples of such particles include smectite groups (for example, synthetic hectorite, synthetic saponite, and natural bentonite), clays (namely fluorine mica), and inorganic particles (namely boehmite).

In the cosmetic of the present invention, it comprise one or more particles selected from smectites, fluorine mica, and boehmite.

The blending quantity of (b) the particles with the average particle size of less than 500 nm is preferably 0.1 to 20 mass % of the total cosmetic. When it is too small, creaming may occur and the viscosity of the emulsion phase alone may increase temporally, allowing the cosmetics to become gel. The blending quantity of component (b) is especially preferably 5 mass % or less. When it is too large, the base material may become too hard.

In the oil-in-water emulsion cosmetics of the present invention, the blending mass ratio of the component (a) and the component (b), i.e., the blending mass of the component (a): blending mass of the component (b) is preferably 1:20 to 2:1. When the blending mass ratio of the component (a) is too small, the emulsion stability may be poor and the base material may become too hard. When the blending mass ratio of the component (b) is too small, creaming may occur and the viscosity of the emulsion phase alone may increase temporally, allowing the cosmetics to become gel.

### ((c) Oil component)

For (c) an oil component that is incorporated in the oil-in-water emulsion cosmetics of the present invention, the oil component usually used in cosmetics can be incorporated in such a range that the stability of the emulsion is not deteriorated.

Examples of liquid oils include silicone oils. Examples of silicone oils include chainlike silicones (for example, polydimethylsiloxane, methylphenylpolysiloxane, and methylhydrogenpolysiloxane) and cyclic silicones (for example, octamethylcyclotetrasiloxane, decamethylcyclopentasiloxane, and dodecamethylcyclohexasiloxane).

Examples of polar oils include ester oils (for example, octyl methoxycinnamate, cetyl octanoate, hexyl laurate, isopropyl myristate, octyl palmitate, isocetyl stearate, isopropyl isostearate, octyl isopalmitate, isodecyl isostearate, 2-ethylhexyl succinate, and diethyl sebacate).

Examples of non-polar oils include hydrocarbon oils (for example, decane, dodecane, liquid paraffin, squalane, squalene, and paraffin).

Examples of solid oils include solid fats (for example, cacao butter, coconut oil, horse fat, hydrogenated coconut oil, palm oil, beef fat, mutton suet, and hydrogenated caster oil), hydrocarbons (for example, paraffin wax (linear hydrocarbon), microcrystalline wax (branched saturated hydrocarbon), ceresin wax, Japan wax, montan wax, and Fischer-Tropsch wax), waxes (for example, beeswax, lanolin, carnauba wax, candelilla wax, rice bran wax (rice wax), spermaceti, jojoba oil, insect wax, kapok wax, bayberry wax, shellac wax, sugarcane wax, lanolin fatty acid isopropyl, hexyl laurate, reduced lanolin, hard lanolin, POE lanolin alcohol ether, POE lanolin alcohol acetate, POE cholesterol ether, lanolin fatty acid polyethylene glycol, and POE hydrogenated lanolin alcohol ether), higher fatty acids (for example, myristic acid, palmitic acid, stearic acid, and behenic acid), and higher alcohols (for example, cetyl alcohol, stearyl alcohol, behenyl alcohol, myristyl alcohol, and cetostearyl alcohols).

The blending quantity of (c) the oil component is preferably 5 to 70 mass % of the total cosmetic. The blending quantity of component (c) is especially preferably 15 mass % or higher. When it is too small, the temporal emulsion stability may be poor. The blending quantity of component (c) is especially preferably 65 mass % or less. When it is too large, the sticky feeling may be generated.

### ((d) Aqueous component)

For (d) an aqueous component that is incorporated in the oil-in-water emulsion cosmetics of the present invention, the aqueous component usually used in cosmetics can be incorporated in such a range that the stability of the emulsion is not deteriorated.

It is preferable that the cosmetic of the present invention comprises water as main component of aqueous component.

Examples of lower alcohols include ethanol, propanol, and isopropanol.

Examples of moisturizers include 1,3-butylene glycol, polyethylene glycol, propylene glycol, dipropylene glycol, hexylene glycol, glycerin, diglycerin, xylitol, maltitol, maltose, D-mannitol, and POE-POP random copolymer.

Examples of UV absorbers include benzoic acid UV absorbers (for example, p-aminobenzoic acid), anthranilic acid UV absorbers (for example, methyl anthranilate), salicylic acid UV absorbers (for example, octyl salicylate and phenyl salicylate), cinnamic acid UV absorbers (for example, isopropyl p-methoxycinnamate, octyl p-methoxycinnamate, and glyceryl mono-2-ethylhexanoate di-p-methoxycinnamate), benzophenone UV absorbers (for example, 2,4-dihydroxybenzophenone, 2-hydroxy-4-methoxybenzophenone, and 2-hydroxy-4-methoxybenzophenone-5-sulfonic acid), urocanic acid, 2-(2'-hydroxy-5'-methylphenyl)benzotriazole, and 4-tert-butyl-4'-methoxybenzoylmethane.

Examples of sequestering agents include sodium edetate, sodium metaphosphate, and phosphoric acid.

Examples of antioxidant agents include ascorbic acid, α-tocopherol, dibutylhydroxytoluene, and butylhydroxyanisol.

Examples of drugs include vitamins (for example, vitamin A oil, retinol, retinol palmitate, inositol, pyridoxine hydrochloride, benzyl nicotinate, nicotinamide, dl-α-tocopherol nicotinate, magnesium ascorbate phosphate, ascorbic acid 2-glucoside, vitamin D2 (ergocalciferol), potassium dl-α-tocopherol 2-L-ascorbate phosphate diester, dl-α-tocopherol, dl-α-tocopheryl acetate, pantothenic acid, and biotin), anti-inflammatory agents (for example, allantoin and azulene), whitening agents (for example, arbutin), astringent agents (for example, tannic acid), sulfur, lysozyme chloride, pyridoxine hydrochloride, γ-orizanol, and tranexamic acid.

The above-mentioned drugs can be used in a free state, a form of acid or basic salt if one can become salts, or a form of ester if one has a carboxylic group.

The blending quantity of (d) the aqueous component is preferably 30 to 95 mass % of the total cosmetic, and especially preferably 35 to 65 mass %.

In the oil-in-water emulsion cosmetic of the present invention, the blending quantity of thickener is preferably 0.05 mass % or less of the total cosmetic, and it is especially preferable to comprise no thickener. The incorporation of a thickener may cause sticky or slimy feelings.

Examples of the thickeners include oil-soluble thickeners corresponding to the component (c) and water-soluble thickeners corresponding to the component (d).

Examples of oil-soluble thickeners include condensation products of dibenzylidene sorbitol, tribenzylidene sorbitol, dibenzylidene xylitol or benzaldehydes with polyol having 5 hydroxyl groups or more, metallic soaps (for example, calcium stearate, calcium palimitate, litium 2-ethylhexanoate, and aluminum 12-hydroxystearate), N-acylamino acid amides (for example, lauroyl glutamate dibutylamide, lauroyl glutamate stearylamide, dicaproyl lysine laurylamine salt, dicaproyl lysine lauryl ester, and dicaproyl lysine lauryl phenylalanine laurylamide), derivatives (for example, ester and amine salt), dextrin fatty acid ester, and 12-hydroxystearic acid.

Examples of water-soluble thickeners include plant-based polymer (for example, gum arabic, tragacanth gum, galactan, guar gum, carrageenan, pectine, quince seed (Cydonia oblonga) extract, brown algae powder, and agar), microorganism-based polymer (for example, xanthan gum, dextran, and pullulan), animal-based polymer (for example, collagen, casein, albumin, and gelatine), starches (for example, carboxymethyl starch, and methylhydroxy starch), celluloses (for example, methylcellulose, nitrocellulose, ethylcellulose, methylhydroxypropylcellulose, hydroxyethylcellulose, cellulose sulfate, hydroxypropylcellulose, carboxymethylcellulose, crystalline cellulose, and cellulose powder), vinyl-based polymer (for example, polyvinyl alcohol, polyvinyl methyl ether, polyvinylpyrrolidone, and carboxyvinylpolymer), acrylic polymer (for example, polyacrylic acid and its salt, and polyacrylimide), glicyrrhizic acid and its salt, and alginic acid and its salt.

In the oil-in-water emulsion cosmetic of the present invention, the components usually used in cosmetics (for example, pH adjuster, preservative, antimicrobial, neutralizer, plant extract, perfume, and dye) can be incorporated in such a range that the effects of the present invention are not deteriorated.

In the oil-in-water emulsion cosmetic of the present invention, the blending quantity of surfactant is less than 0.5 mass % of the total cosmetic, and it is especially preferable to comprise no surfactant. The incorporation of a surfactant may cause stickiness or may cause skin irritation in extremely rare cases.

Examples of surfactants include glyceryl fatty acid esters, polyglyceryl fatty acid esters, propylene glycol fatty acid esters, POE-sorbitan fatty acid esters, POE-sorbit fatty acid esters, POE-glyceryl fatty acid esters, POE-fatty acid esters, POE-alkyl ethers, POE-alkyl phenyl ethers, POE/POP-alkyl ethers, POE-castor oil derivatives, POE-hydrogenated castor oil derivatives, POE-beeswax/lanoline derivatives, alkanolamides, POE-propyleneglycol fatty acid esters, POE-alkyl amines, and POE-fatty acid amides.

The oil-in-water emulsion cosmetic of the present invention can take any of various product forms, and the examples include milky lotion, cream, foundation, sunscreen, and makeup base.

### EXAMPLES

Hereinafter, the present invention will be more concretely described by examples. However, the present invention is not limited by these examples. The blending quantity is expressed in mass % unless otherwise noted.

In certain test examples and formulation exmples an amphiphatic protein is used as an amphipathic substance instead of a copolymer of 2-acrylamido-2methylpropane sulfonic acid or a salt thereof and vinylpyrrolidone according to the present invention. These test examples and formulation examples are given for reference but are out of scope of protection.

At first, the evaluation methods used in the present invention will be described.

### Evaluation (1): Average particle size of the emulsified particles

The average particle size of each sample (emulsion particle) immediately after the preparation was measured with a Zetasizer (Zetasizer Nano ZS, manufactured by SYSMEX CORPORATION).

### Evaluation (2): Viscosity

The viscosity of each sample (stored at 25 °C) immediately or one week after the preparation was measured with a B-type viscometer (BL-type, 12 rpm).

### Evaluation (3): Stability

The appearance of each sample immediately or one week after the preparation was observed (stored at 25 °C).
A: The sample was homogeneously liquid form.
B: Creaming (separation without coalescence of emulsion particles) was found in the sample.
C: Oil floating was observed for the sample.
D: The sample separated.

### Evaluation (4): Feeling in use

40 professional panelists conducted test by applying each sample to face and evaluated the feeling in use (absence of an oily feeling, absence of a slimy feeling during the application, and absence of stickiness after the application) based on six evaluation criteria (remarkably effective, effective, slightly effective, between effective and ineffective, slightly ineffective, and ineffective).

### (Evaluation method)

A: The ratio of the professional panelists who gave the evaluation of remarkably effective, effective or slightly effective was 50 % or higher.
B: The ratio of the professional panelists who gave the evaluation of remarkably effective, effective or slightly effective was 30 to less than 50 %.
C: The ratio of the professional panelists who gave the evaluation of remarkably effective, effective or slightly effective was less than 30 %.

At first, Pickering emulsions using amphipathic protein are investigated. The present inventors prepared each oil-in-water emulsion with the blending composition shown in Table 1 in a normal method. Then, each emulsion was evaluated for the above-described evaluation criteria (1) to (3). The results are shown in Table 1. The test examples 1-1 to 1-3 are not covered by the claims of the present invention.

**[Table 1]**

| Test Example | 1-1 | 1-2 | 1-3 |
|---|---|---|---|
| Wheat-derived protein (*1) | 0.5 | - | 0.5 |
| Barley-derived protein (*2) | - | 0.5 | - |
| Liquid paraffin | 35 | 35 | 65 |
| Water | balance | balance | balance |
| Average emulsion particle size (*µ* m) | 12 | 14 | 15 |
| Viscosity immediately after the preparation (mPa▪s) | - | - | 6800 |
| Viscosity one week after the preparation (mPa▪s) | - | - | 22000 |
| Appearance immediately after the preparation | B | B | A |
| Appearance one week after the preparation | gel (*3) | gel(*3) | gel |

| | | | |
|---|---|---|---|
| (*1): Plantasol W (manufactured by Gelita) (*2): Beer-derived (Security name: Scherdel Helle Weisse) (*3): The emulsion phase was gelation. | | | |

According to Test Example 1-1 and Test Example 1-2, with the use of wheat-derived protein, or barley-derived protein, a Pickering emulsion with a small emulsion particle size can be prepared; however, creaming is observed and gelation takes place with time.

According to Test Example 1-3, a stable emulsion can be produced immediately after preparation by increasing the blending quantity of oil; however, gelation also takes place with time.

Thus, a Pickering emulsion can be produced without the use of a surfactant by blending an amphipathic protein. However, the protein forms a network with time and thickens; therefore, it is difficult to obtain a stable emulsion.

Amphipathic-protein-like components, with which a stable and fine Pickering emulsion can be formed in the present invention system, were investigated. The present inventors prepared each oil-in-water emulsion with the blending composition shown in Table 2 in a normal method. Then, each emulsion was evaluated for the above-described evaluation criteria (1) to (3). The results are shown in Table 2. The examples 3-2 to 3-4 and 3-6, 3-7 are not covered by the present invention.

**[Table 2]**

| Test Example | 3-1 | 3-2 | 3-3 | 3-4 | 3-5 | 3-6 | 3-7 |
|---|---|---|---|---|---|---|---|
| Vinylpyrrolidone/2-acrylamido-2-methylpropane sulfonic acid (salt) copolymer (*5) | 0.1 | - | - | - | 0.1 | - | - |
| Dimethylacrylamide/2-acrylamido-2-methylpropane sulfonic acid crosspolymer (*6) | - | 0.1 | - | - | - | 0.1 | - |
| Acrylic acid-methacrylic acid alkyl ester copolymer (*7) | - | - | 0.1 | - | - | - | 0.1 |
| Synthetic hectorite (*4) | 1 | 1 | 1 | 1 | - | - | - |
| Liquid paraffin | 35 | 35 | 35 | 35 | 35 | 35 | 35 |

| Water | balance | balance | balance | balance | balance | balance | balance |
|---|---|---|---|---|---|---|---|
| Average emulsion particle size (*µ* m) | ∼1(5) | ∼0.5 | ∼1(5) | ∼ 2.5(5) | ∼ 5(7.5) | ∼ 0.5(5) | ∼1(10) |
| Viscosity immediately after the preparation (mPa▪s) | 5940 | 4400 | 420 | 3630 | - | - | - |
| Viscosity one week after the (mPa▪s) | 5450 | 4160 | 400 | 3640 | - | - | - |
| Appearance immediately after the | A | A | A | C | C | C | C |
| Appearance one week after the | A | A | B | C | B | B | B |

| | | | | | | | |
|---|---|---|---|---|---|---|---|
| (*5): Aristoflex AVC (manufactured by Clariant UK) (*6): SUpolymer G-1 (manufactured by TOHO Chemical Industry Co., Ltd.) (*7): PEMULEN TR-2 (manufactured by Lubrizol Advanced Materials) | | | | | | | |

According to Test Example 3-1 and Test Example 3-2, when components (b) to (d) and vinylpyrrolidone/2-acrylamido-2-methylpropane sulfonic acid (salt) copolymer or dimethylacrylamide/2-acrylamido-2-methylpropane sulfonic acid crosspolymer, which is an amphipathic substance, are blended in the present invention, an emulsion with excellent emulsion stability and a small emulsion particle size can be obtained.

According to Test Examples 3-3 to 3-7, when only vinylpyrrolidone/2-acrylamido-2-methylpropane sulfonic acid (salt) copolymer or only dimethylacrylamide/2-acrylamido-2-methylpropane sulfonic acid crosspolymer is used and when the conventional surfactant and component (b) are used, a stable and fine emulsion cannot be obtained.

Accordingly, it is clarified that (a) vinylpyrrolidone/2-acrylamido-2-methylpropane sulfonic acid (salt) copolymer and - not covered by the present invention - dimethylacrylamide/2-acrylamido-2-methylpropane sulfonic acid crosspolymer can be used as (a) amphipathic substance other than amphipathic protein.

As a result of further investigation by the present inventors, component (a) represented by vinylpyrrolidone/2-acrylamido-2-methylpropane sulfonic acid (salt) copolymer needs to be a copolymer of 2-acrylamido-2methylpropane sulfonic acid or a salt thereof and vinylpyrrolidone.

Subsequently, the present inventors further studied oil-in-water emulsion cosmetics according to comparative examples not being covered ba the scope of the present invention. The present inventors prepared each oil-in-water emulsion with the blending composition shown in Table 3 in a normal method. Then, each emulsion was evaluated for the above-described evaluation criteria (2) and (4). The results are shown in Table 3. Test examples 2-1 and 4-1 are not covered by the scope of the present invention.

**[Table 3]**

| Test Example | 2-1 | 4-1 |
|---|---|---|
| Wheat-derived protein (*1) | 0.5 | 0.5 |
| Synthetic hectorite (*4) | 0.5 | - |
| Carboxyvinylpolymer | - | 0.1 |
| Pottasium hydroxide | - | 0.03 |
| Liquid paraffin | 35 | 35 |
| Water | balance | balance |
| Viscosity immediately after the (mPa▪s) | 4200 | 5800 |
| Absence of an oily feeling | A | B |
| Absence of a slimy feeling during the application | A | C |
| Absence of stickiness after the application | A | C |

From Table 3, the sample of Test Example 2-1 is excellent in the feeling in use.

On the other hand, the sample of Test Example 4-1, wherein the stabilization was attempted by thickening the water phase, was poor in the feeling in use.

Accordingly, in the oil-in-water emulsion cosmetic of the present invention, the blending quantity of thickener is preferably 0.05 mass % or less, and it is more preferable to comprise no thickener.

In the following, formulation examples of the oil-in-water emulsion cosmetic are listed, wherein wheat-, barley- or soybean derived protein or casein are used instead of copolymer (a) according to the present invention. Accordingly, formulation examples 1 to 5 are not coverd by the scope of the present invention.

### Formulation Example 1 (Example out of the scope of protection): Moisturizing milky lotion

| Wheat-derived protein (*1) | 0.5 mass % |
|---|---|
| Silicon oil | 10 |
| Liquid paraffin | 20 |
| Synthetic hectorite (*4) | 1 |
| Phenoxyethanol | 0.3 |
| Dynamite glycerin | 4 |
| 1,3-butylene glycol | 5 |
| Polyethylene glycol | 3 |
| Water | balance |

### Formulation Example 2 (Example out of the scope of protection): Sunscreen milky lotion

| Barley-derived protein (*2) | 0.5 mass % |
|---|---|
| Volatile silicon oil | 10 |
| Isononyl isononate | 4 |
| Octyl methoxycinnamate | 8 |
| Butyl methoxy dibenzoylmethane | 4 |
| Hydrophobized microparticle zinc oxide | 4 |
| Microparticle titanium oxide | 2 |
| Boemite | 0.5 |
| Phenoxyethanol | 5 |
| Dipropylene glycol | 3 |
| Water | balance |

### Formulation Example 3 (Example out of the scope of protection): Whitening milky lotion

| Casein | 0.5 mass % |
|---|---|
| Silicon oil | 5 |
| Ethyl 2-ethylhexanoate | 8 |
| α-olefin oligomer | 10 |
| Synthetic saponite (Smecton SA) | 1 |
| Phenoxyethanol | 0.3 |
| Dynamite glycerin | 4 |
| POE/POP random copolymer | 4 |
| Tranexamic acid | 3 |
| Ascorbic acid | 3 |
| Water | balance |

### Formulation Example 4 (Example out of the scope of protection): Moisturing cream

| Wheat-derived protein (*1) | 0.5 mass % |
|---|---|
| Squaran | 8 |
| Jojoba oil | 5 |
| Pentaerythritol tetra 2-ethylhexanoate | 8 |
| Vaseline | 3 |
| Palm hydrogenated oil | 2 |
| Spherical silica | 3 |
| Phenoxyethanol | 0.3 |
| Dynamite glycerin | 4 |
| POE/POP random copolymer | 4 |
| POE (60) glyceryl monoisostearate | 0.1 |

| | |
|---|---|
| Citric acid | 0.03 |
| Sodium citrate | 0.07 |
| Water | balance |

### Formulation Example 5 (Example out of the scope of protection): Moisturing milky lotion

| Soybean-derived protein | 0.5 mass % |
|---|---|
| Isohexadecane | 10 |
| Octyl palmitate | 20 |
| Fluorine mica | 1 |
| Phenoxyethanol | 0.3 |
| Dynamite glycerin | 4 |
| Erythritol | 2 |
| Polyethyleneglycol | 3 |
| Water | balance |

## Claims

1. An oil-in-water emulsion cosmetic comprising the following components (a) to (d):
(a) an amphipathic substance being a copolymer of 2-acrylamido-2methylpropane sulfonic acid or a salt thereof and vinylpyrrolidone,
(b) particles with the average particle size of less than 500 nm, wherein component (b) is selected from smectites, fluorine mica and boehmite,
(c) an oil component, and
(d) an aqueous component,
wherein the blending quantity of a surfactant is less than 0.5 mass % of the total amount of the cosmetic.

2. The oil-in-water emulsion cosmetic according to claim 1, wherein component (a) is represented by the following general formula (1). 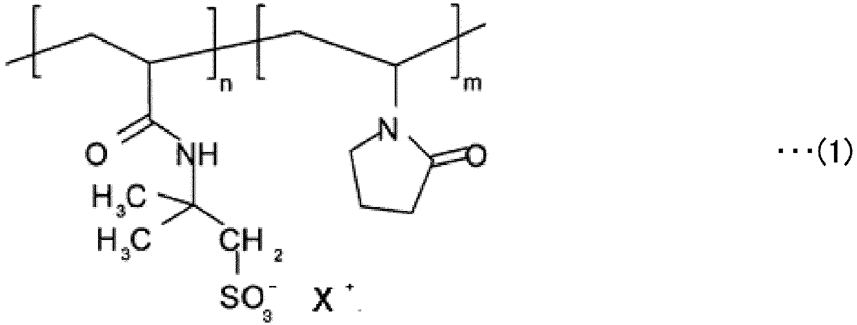 (In the formula, m and n are average addition mole numbers, and X⁺ represents a proton, an alkali metal cation, an alkaline earth metal cation, an ammonium ion, or an organic cation.)

3. The oil-in-water emulsion cosmetic according to claim 1 or 2, wherein the blending quantity of component (c) is 5 to 70 mass % of the total amount of the cosmetic.

4. The oil-in-water emulsion cosmetic according to any of claims 1 to 3, wherein the blending mass ratio of component (a) and component (b) is 1:20 to 2:1.

5. The oil-in-water emulsion cosmetic according to any of claims 1 to 4, wherein the blending quantity of a thickener is 0.05 mass % or less of the total amount of the cosmetic.

## Patentansprüche

1. Öl-in-Wasser-Emulsionskosmetikum, umfassend die folgenden Komponenten (a) bis (d):
(a) eine amphipathische Substanz, bei der es sich um ein Copolymer aus 2-Acrylamido-2-methylpropansulfonsäure oder einem Salz davon und Vinylpyrrolidon handelt,
(b) Partikel mit einer mittleren Partikelgröße von weniger als 500 nm, wobei Komponente (b) ausgewählt ist aus Smektiten, Fluorglimmer und Böhmit,
(c) eine Ölkomponente und
(d) eine wässrige Komponente,
wobei die Beimischungsmenge eines Tensids weniger als 0,5 Masse-% der Gesamtmenge des Kosmetikums beträgt.

2. Öl-in-Wasser-Emulsionskosmetikum nach Anspruch 1, wobei Komponente (a) durch die folgende allgemeine Formel (1) wiedergegeben ist: 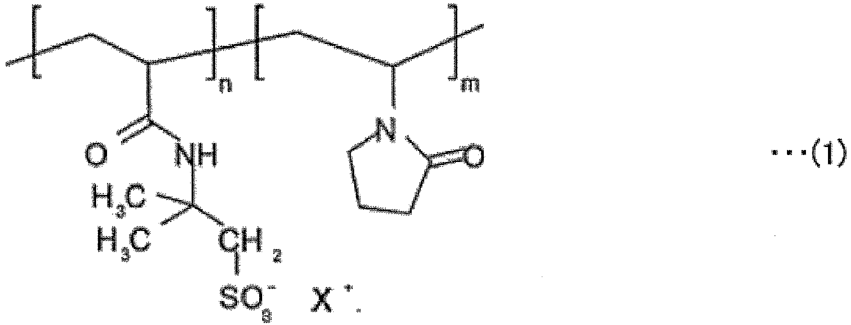 (In der Formel sind m und n mittlere Zugabemolzahlen und steht X⁺ für ein Proton, ein Alkalimetallkation, ein Erdalkalimetallkation, ein Ammoniumion oder ein organisches Kation.)

3. Öl-in-Wasser-Emulsionskosmetikum nach Anspruch 1 oder 2, wobei die Beimischungsmenge von Komponente (c) 5 bis 70 Masse-% der Gesamtmenge des Kosmetikums beträgt.

4. Öl-in-Wasser-Emulsionskosmetikum nach einem der Ansprüche 1 bis 3, wobei das Mischungsmasseverhältnis von Komponente (a) und Komponente (b) 1:20 bis 2:1 beträgt.

5. Öl-in-Wasser-Emulsionskosmetikum nach einem der Ansprüche 1 bis 4, wobei die Beimischungsmenge eines Verdickungsmittels 0,05 Masse-% oder weniger der Gesamtmenge des Kosmetikums beträgt.

## Revendications

1. Cosmétique sous forme d'émulsion huile-dans-eau comprenant les composants (a) à (d) suivants :
(a) une substance amphipathique qui est un copolymère d'acide 2-acrylamido-2-méthylpropane sulfonique ou un sel de celui-ci et de vinylpyrrolidone,
(b) des particules ayant la dimension moyenne des particules de moins de 500 nm, le composant (b) étant sélectionné parmi des smectites, du mica fluoré et de la boehmite,
(c) un composant huileux, et
(d) un composant aqueux,
dans lequel la quantité de mélange d'un tensioactif est de moins de 0,5 % en masse de la quantité totale du cosmétique.

2. Cosmétique sous forme d'émulsion huile-dans-eau selon la revendication 1, le composant (a) est représenté par la formule générale (1) suivante : 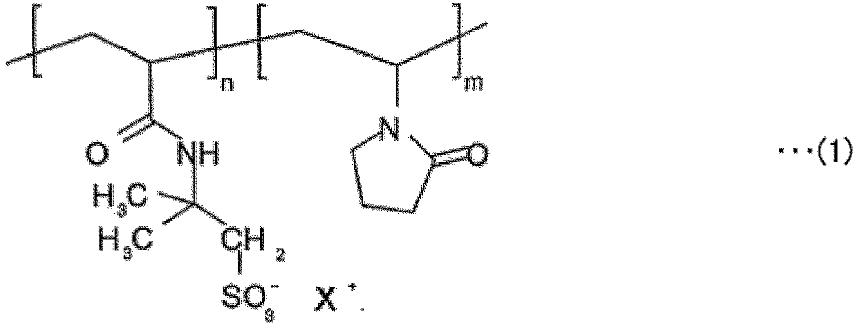 (dans la formule, m et n sont des nombres de moles moyens d'addition, et X⁺ représente un proton, un cation de métal alcalin, un cation de métal alcalino-terreux, un ion ammonium, ou un cation organique).

3. Cosmétique sous forme d'émulsion huile-dans-eau selon la revendication 1 ou 2, dans lequel la quantité de mélange du composant (c) est de 5 à 70 % en masse de la quantité totale du cosmétique.

4. Cosmétique sous forme d'émulsion huile-dans-eau selon l'une quelconque des revendications 1 à 3, dans lequel le rapport en masse de mélange entre composant (a) et composant (b) est de 1 : 20 à 2 : 1.

5. Cosmétique sous forme d'émulsion huile-dans-eau selon l'une quelconque des revendications 1 à 4, dans lequel la quantité de mélange d'un épaississant est de 0,05 % en masse ou moins de la quantité totale du cosmétique.
